Europäisches Patentamt
European Patent Office
Office européen des brevets

(11) Publication number: 0 045 220 B1

(12) EUROPEAN PATENT SPECIFICATION

(45) Date of publication of patent specification: 06.11.85

(51) Int. Cl.[4]: C 12 Q 1/28, G 01 N 33/52

(21) Application number: 81303495.6

(22) Date of filing: 30.07.81

# (54) Method and test composition for determination of hydrogen peroxide.

(30) Priority: 30.07.80 JP 103665/80

(43) Date of publication of application: 03.02.82 Bulletin 82/05

(45) Publication of the grant of the patent: 06.11.85 Bulletin 85/45

(84) Designated Contracting States: DE FR GB IT

(56) References cited:
DE-A-2 110 342
GB-A-1 299 845
GB-A-1 400 897
GB-A-1 473 945
GB-A-2 002 517
US-A-3 654 179
US-A-3 791 988

CHEMICAL ABSTRACTS, vol. 80, nr. 12, March 25, 1974, page 482 abstract 66505b Columbus, Ohio, US H. PUZANOWSKA-TARASIEWICZ: "Phenothiazine derivatives as new indicators in chemical analysis. III. Bromatometric determination of hydroquinone and ascorbic acid"

(73) Proprietor: KYOWA HAKKO KOGYO CO., LTD
Ohtemachi Bldg. Ohtemachi 1-chome Chiyoda-ku
Tokyo (JP)

(72) Inventor: Miike, Akira
410-1 Nameri Nagaizumi-cho
Sunto-gun Shizuoka-ken (JP)
Inventor: Shimizu, Yoshiaki
127-5 Shimonagakubo Ikeda Nagaizumi-cho
Sunto-gun Shizuoka-ken (JP)
Inventor: Tatano, Toshio
2297-6, Ooka
Numazu-shi Shizuoka-ken (JP)
Inventor: Watanabe, Katsuyuki
3-19-20, Fujimi-cho
Higashimurayama-shi Tokyo (JP)

(74) Representative: Lambert, Hugh Richmond et al
D. YOUNG & CO. 10 Staple Inn
London, WC1V 7RD (GB)

(56) References cited:
CHEMICAL ABSTRACTS, vol. 89, no. 14, October 2, 1978, page 888, abstract 122422x Columbus, Ohio, US N.V. RAO et al.: "Oxazine dyes as new iodometric indicators"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 045 220 B1

**Description**

The present invention provides a method and a test composition for the determination of hydrogen peroxide using a novel chromogen as a hydrogen donor. More particularly, it provides a method for the determination of hydrogen peroxide by reacting hydrogen peroxide with the chromogen in the presence of peroxidase and determining the pigment formed, and test compositions suitable for the determination.

Heretofore, the determination of a substrate was generally carried out by oxidizing the substrate by the action of oxidase and determining the formed hydrogen peroxide. For example, uric acid is oxidized by uricase and cholesterol is oxidized by cholesterol oxidase to form hydrogen peroxide. The hydrogen peroxide is determined by reacting the hydrogen peroxide with a chromogen in the presence of peroxidase to form a pigment and measuring the absorbancy of the reaction solution coloured by the formation of the pigment in the visible ray region. In such determinations various chromogens have been proposed such as 4-aminoantipyrine (hereinafter referred to as "4AA") and phenol, 4AA and N,N-dimethylaniline, 4AA and N-ethyl-N-(β-hydroxyethyl)-m-toluidine, 3-methylbenzothiazolin hydrazone, N,N-diethylaniline and 4,4'-bis(dimethylamino)diphenylamine, otherwise known as Bindschedler's Green, see in particular U.S.—A—3,654,179. Whilst such chromogens function satisfactorily, there is still a demand for chromogens which are of superior stability and colour and which are largely insensitive to other components which may be present in the samples under test including such in vivo components as haemoglobin, bilirubin and glutathione.

In accordance with the present invention we have found that compounds of the formulae I and II, indicated below, are excellent chromogens in the colorimetric estimation of hydrogen peroxide involving reaction of the hydrogen peroxide with peroxidase in the presence of the chromogen:

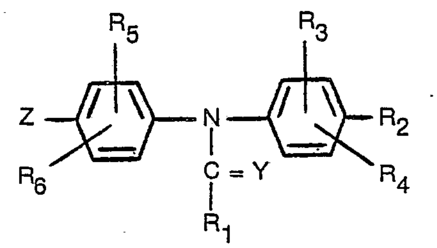

[I]

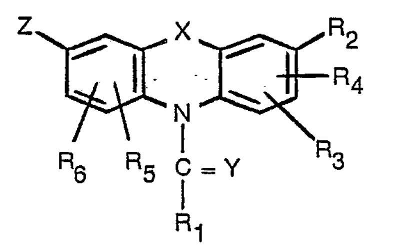

[II]

In the formulae, Z represents hydroxyl or substituted or unsubstituted amino, Y represents an oxygen atom or sulphur atom, $R_1$ represents hydrogen, alkyl, alkenyl, aryl, amino or mono- substituted amino, $R_2$ represents hydrogen, hydroxyl, alkyl, aryl, alkenyl, amino, alkylamino or alkoxy, $R_3$, $R_4$, $R_5$ and $R_6$ represent hydrogen, alkyl, acyl, aryl, halogen, alkenyl, nitro, sulfo, carboxyl, hydroxyl, hydroxylalkyl or alkoxy, $R_3$ and $R_4$ or $R_5$ and $R_6$ may form alkenylene,

X represents —S—, —O—, 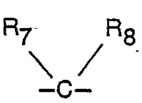 or 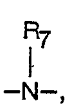,

$R_7$ and $R_8$ represent hydrogen, alkyl, alkenyl or aryl.

In the above definitions the following meanings apply:

in the definition of Z, substituted amino means mono- or di-substituted amino wherein the substituent(s) is or are alkyl, alkenyl, hydroxyalkyl, aryl, or acylalkyl, such terms being further qualified below;

in the definition of $R_1$, mono-substituted amino means amino substituted by alkyl, substituted alkyl, alkenyl, phenyl, naphthyl or substituted phenyl, such terms being further qualified below;

"alkyl" means $C_1$—$C_4$ alkyl e.g. methyl, ethyl, propyl or butyl;

"alkenyl" means $C_2$—$C_5$ alkenyl, e.g. vinyl, propenyl or butenyl;

"alkoxy" means $C_1$—$C_4$ alkoxy, e.g. methoxy, ethoxy, propoxy or butoxy;

"acyl" means $C_2$—$C_5$ acyl, e.g. acetyl, propionyl or butyryl;

"aryl" means phenyl, benzyl, napthyl or substituted phenyl;

"substituted phenyl" means phenyl substituted by up to five substituents selected from $C_1$—$C_4$ alkyl, halogen, e.g. chloro or bromo, amino, ($C_1$—$C_4$)alkoxycarbonylamino, ($C_1$—$C_4$)alkoxycarbonylamino-($C_1$—$C_4$)alkyl, alkoxy or acyl (as defined above);

"alkenylene" means $C_3$—$C_4$ alkenylene e.g. $—CH=CH—CH=CH—$ or $—CH=CH—CH_2—$; and

"substituted alkyl" means $C_1$—$C_4$ alkyl substituted by ($C_1$—$C_4$)alkoxycarbonylamino, phenyl or substituted phenyl (as defined above). These compounds are generally known and are easily prepared by the methods illustrated by the following reaction formulae.

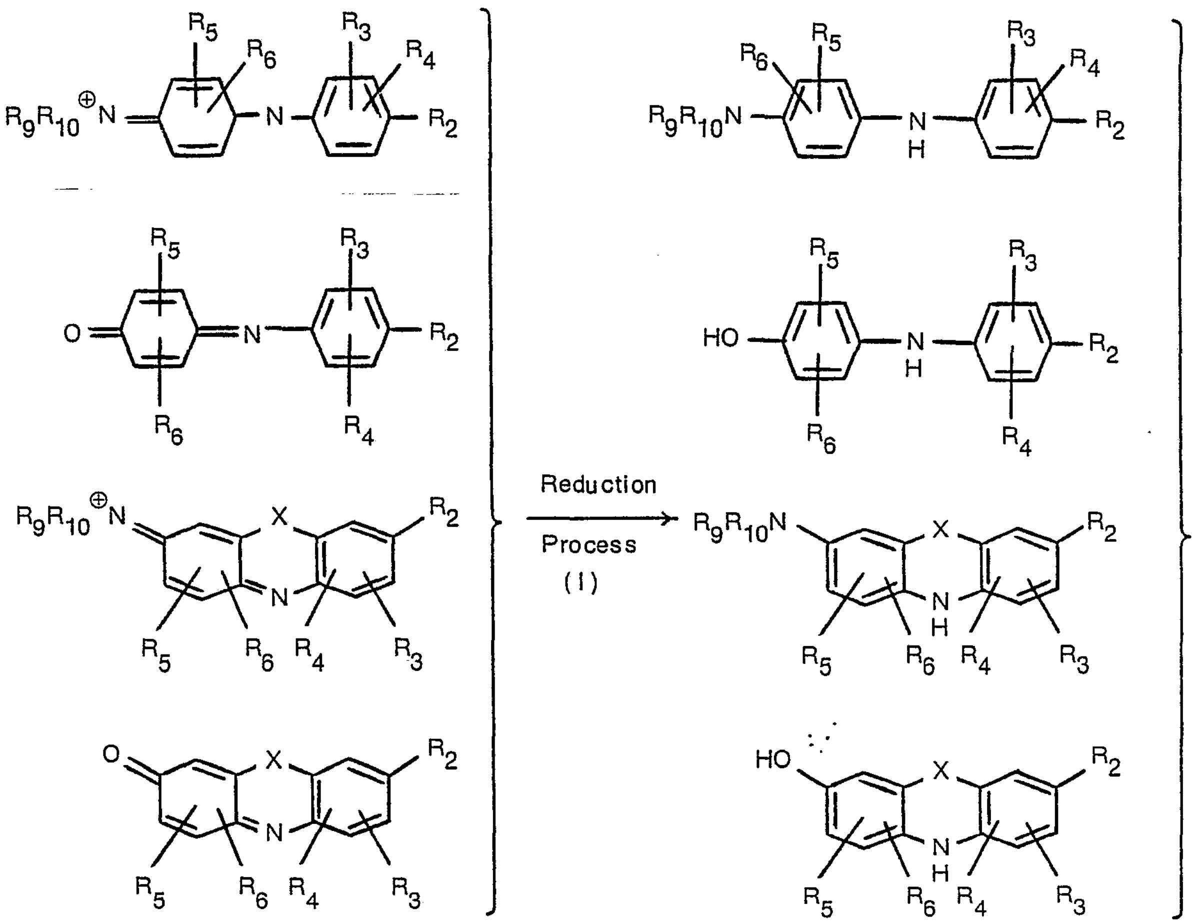

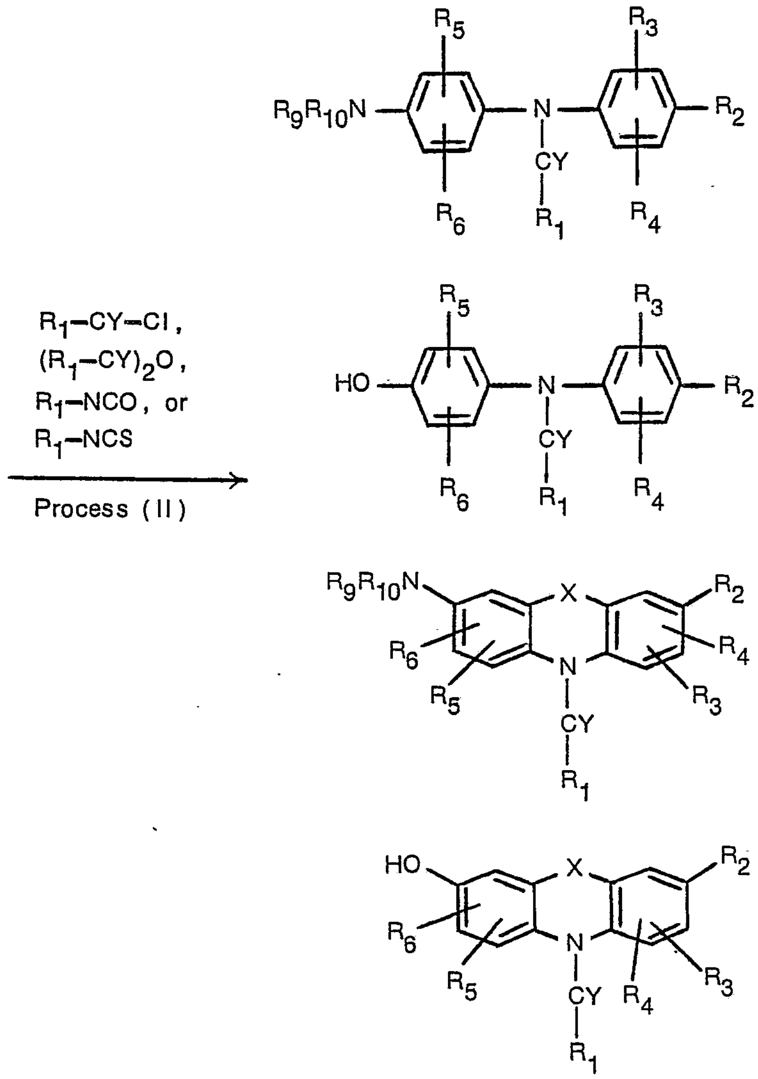


In the formulae, $R_1$—$R_6$, $R_9$, $R_{10}$, X and Y have the same significance as defined above.

The raw materials are known pigments.

In the Process (I), the raw materials is subjected to reduction in the presence of reducing agents such as $NaBH_4$, Fe—HCl, etc. The reduction is carried out in a solvent such as water, methanol, acetone, etc. at from room temperature to the boiling point of the solvent used. After the raw materials are decolorized, the reduction product is isolated from the reaction mixture. To the isolated reduction product or the reaction mixture is added an acylating agent, an alkylthiocarbonylating agent, ester of isocyanic acid, or ester of isothiocyanic acid. The reaction is usually carried out at from room temperature to the boiling point of the solvent used.

The isolation of the desired product from the reaction mixture is carried out by a suitable organic synthesis isolation means such as extraction, concentration, chromatography, recrystallization, filtration, etc.

Examples of chromogens used in the present invention are shown in Table 1.

Symbols "I" or "II" in Table 1 mean compounds of formula (I) or (II) [hereinafter referred to as compound (I) or (II), respectively].

TABLE I

| Compound No. | Z | X | Y | $R_1$ | $R_2$ | $R_3$ | $R_4$ | $R_5$ | $R_6$ |
|---|---|---|---|---|---|---|---|---|---|
| I–1 | $N(CH_3)_2$ | – | S | $NHCH_3$ | $N(CH_3)_2$ | H | H | H | H |
| I–2 | $N(CH_3)_2$ | – | O | 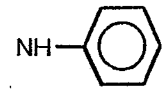 | $N(CH_3)_2$ | H | H | H | H |
| I–3 | $N(CH_3)_2$ | – | O | 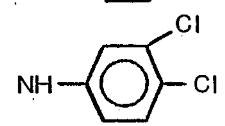 | $N(CH_3)_2$ | H | H | H | H |
| II–4 | $N(CH_3)_2$ | S | O | $NH{-}(CH_2)_6{\cdot}NHCOOCH_3$ | $N(CH_3)_2$ | H | H | H | H |
| II–5 | $N(CH_3)_2$ | S | O | 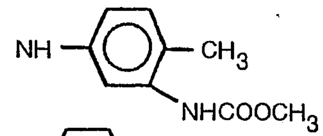 | $N(CH_3)_2$ | H | H | H | H |
| II–6 | $N(CH_3)_2$ | S | O | 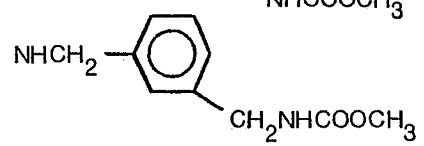 | $N(CH_3)_2$ | H | H | H | H |
| II–7 | $N(CH_3)_2$ | S | O | 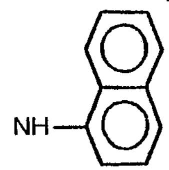 | $N(CH_3)_2$ | H | H | H | H |
| II–8 | $N(CH_3)_2$ | S | O | $CH_3$ | $N(CH_3)_2$ | H | H | H | H |
| II–9 | $N(CH_3)_2$ | S | O | 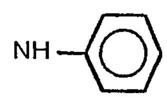 | $N(CH_3)_2$ | H | H | H | H |
| II–10 | $N(CH_3)_2$ | O | O | 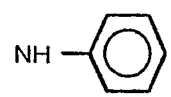 | H | H | H | A | |

A: $-CH{=}CH{-}CH{=}CH-$

The method of the present invention is based on the fact that the reaction of hydrogen peroxide with the present chromogen proceeds stoichiometrically to form a pigment and the amount of pigment formed is proportional to the amount of hydrogen peroxide in the sample.

The principle is illustrated as follows.

Cases where Z is amino or substituted amino

POD means peroxidase.

[I–A]

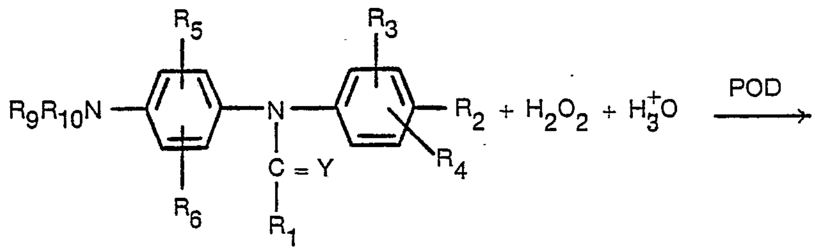

[I–A′]

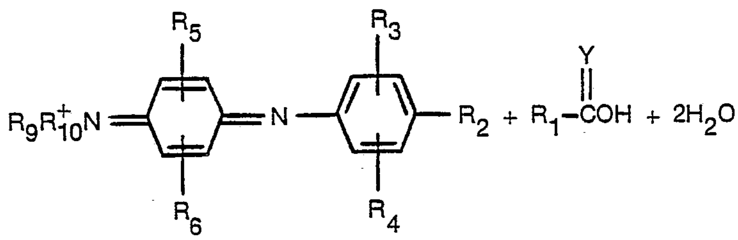

[II–A]

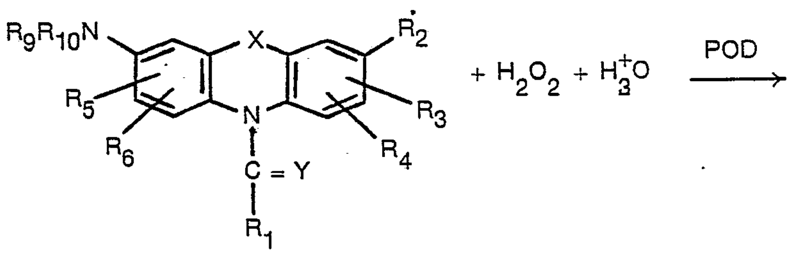

[II–A′]

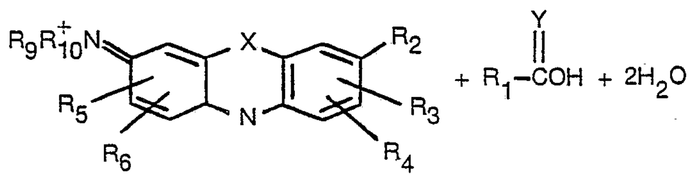

Cases where Z is OH

[I–B]

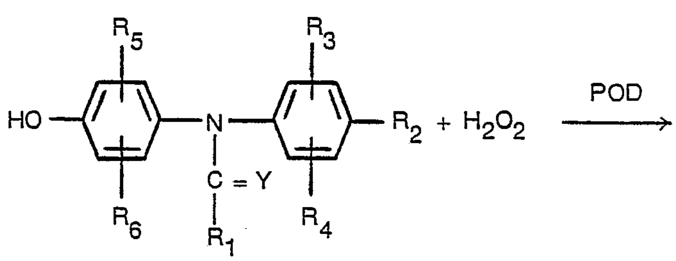

[I–B′]

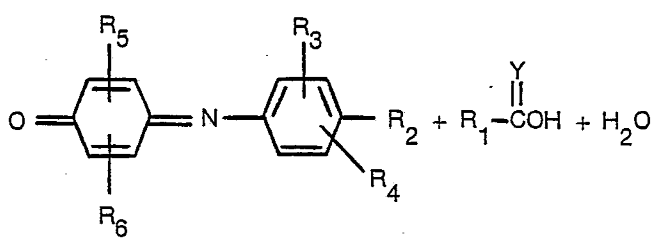

[II–B]

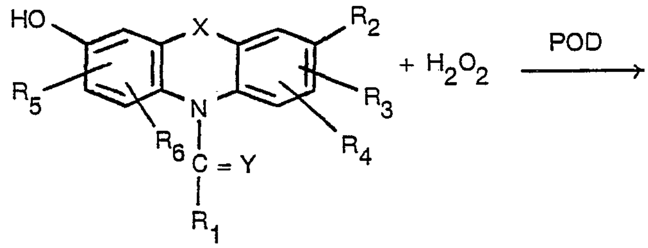


[II–B′]

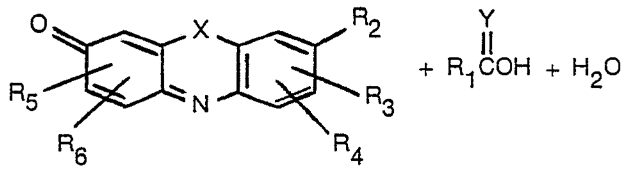

Compounds [I—A′], [I—B′], [II—A′] and [II—B′] formed in the above reactions are known pigments such as Bindschedler's Green, indophenol, Methylene Blue, etc. These pigments have excellent stability and are therefore suitable for the determination of hydrogen peroxide by colorimetry of the solution coloured by the formation of pigment.

Comparative tests between the compounds indicated in Table 1 and known compounds in respect of the degree of colour development and the stability of the colour formed in the reaction with hydrogen peroxide are conducted according to the following method.

A test solution containing 0.1 mg/ml compounds indicated in Table 1, 10 IU/ml POD and if necessary, 0.1% surfactant in a phosphate buffer solution (pH 6.0) is prepared. To 3 ml of the test solution is added 20 µl hydrogen peroxide solution and the absorbancy of the coloured reaction solution (E) is measured at maximum absorption wave-length (λ max) of the pigment formed.

The blank absorbancy ($E_B$) is measured by repeating the same procedures as described above except that water instead of hydrogen peroxide solution is used.

The degree of the colour development ($E—E_B$) of the test compound is calculated defining the degree of colour development ($E—E_B$)′ of 4AA-phenol ($A_1$) as 100.

For comparison, 4AA-dimethylaniline ($A_2$) and 4AA-diethylaniline ($A_3$) are used as chromogens.

The stability of colour is determined as follows. The value of ($E—E_B$) immediately after the reaction is discontinued (in about 30 minutes) is measured. Then the reaction solution is further incubated at 37°C for 4 hours and then ($E—E_B$) is calculated. The symbol "+" means that the decrease of the value of $E—E_B$ is 0—5% during the further incubation and "+" means that the decrease is 5—10%.

The results are shown in Table 2.

TABLE 2

| Chromogen | λ max(nm) | Degree of color | Stability |
|---|---|---|---|
| $A_1$ | 505 | 100 | ± |
| A2 | 550 | 160 | + |
| $A_3$ | 550 | 180 | + |
| I–1 | 728 | 1109 | ± |
| I–2 | 728 | 1022 | ± |
| I–3 | 728 | 981 | ± |
| II–4 | 665 | 1016 | ± |
| II–5 | 665 | 1077 | ± |
| II–6 | 665 | 1054 | ± |
| II–7 | 665 | 962 | ± |
| II–8 | 665 | 1183 | ± |
| II–9 | 665 | 1140 | ± |
| II–10 | 568 | 947 | ± |

When the compound (I) or (II) is used as a chromogen, the maximum absorption wavelength in the visible ray region of the pigment is larger than that of hemoglobin contained in serum, which is around 400 nm and therefore, the absorbancy by the present method is not affected by hemoglobin.

Further, when a turbid sample such as serum containing a large amount of lipid is used, the turbidity causes an error in the absorbancy. The change of absorbancy due to the turbidity becomes large as the wavelength is shortened and therefore, when the compound (I) or (II) is used, the absorbancy is not affected by the turbidity.

In carrying out the determination of hydrogen peroxide according to the present invention, the compound (I) or (II) and POD may be added to the system where hydrogen peroxide is produced (hereinafter referred to as "$H_2O_2$-producing system"). The absorbancy of the reaction solution coloured by the formation of pigment is measured in the visible ray region, 400—760 nm. On the other hand, the standard curve showing the relation between the amount of hydrogen peroxide and absorbancy is prepared by using a standard hydrogen peroxide solution as the sample. The amount of hydrogen peroxide in the sample is calculated by applying the absorbancy obtained to the standard curve.

The reaction is usually carried out at a temperature of 5—50°C, preferably 25—40°C, in a buffer solution having a pH of 2—10 and completed in several minutes.

The chromogen should be used in an equimolar amount with hydrogen peroxide or more, preferably 10—1000 mole equivalents. Peroxidase may be used in a concentration of 0.1—1000 IU/ml.

As buffers, phosphate buffer, tris-HCl buffer, succinate buffer, citrate buffer, acetate buffer, etc. may be used in a concentration of 0.005—2 mol/l.

The present method may be used for the determination of reactants in a system where hydrogen peroxide is produced. Particularly, when the system is an enzymatic reaction, both the $H_2O_2$-producing system and the system where pigment is produced [hereinafter referred to as pigment-producing system] proceed at the same time and therefore, such a method is simple and convenient.

Such an enzymatic reaction includes the combination of oxidase and a substrate thereof and examples of the combination are uric acid-uricase, cholesterol-cholesterol oxidase, cholesterol estercholesterol esterase and cholesterol oxidase, xanthin, hypoxanthin or guanine-xanthin oxidase, phospholipase D-lecitin-choline oxidase, choline-choline oxidase, pyruvic acid-pyruvate oxidase-phosphoric acid, triglyceride-lipoprotein lipase-ATP-glycerinkinase-glycerin-3-phosphate oxidase, fatty acid-coenzyme A-acyl Co. A synthetase-acyl Co A oxidase, triglyceride-lipase-glycerol oxidase, glucose-glucose oxidase and galactose-galactose oxidase.

The substrates of these enzymatic reactions are contained in serum, urine, etc. and the determination of the substrates is useful for diagnostic purposes.

The hydrogen peroxide-producing reaction and the pigment-producing reaction may be conducted stepwise and preferably, the determination of hydrogen peroxide is performed by adding to the sample the components necessary for the determination of hydrogen peroxide, conducting all the reactions in one step and measuring the absorbancy of the reaction solution.

The components comprise oxidase for the substrate to be determined, peroxidase, compound (I) or (II), buffer solution and if necessary a surfactant.

If components for oxidizing the substrate in addition to oxidase for the substrate are required, the components must be added to $H_2O_2$-producing system.

Another aspect of the present invention is to provide a test composition for the determination of hydrogen peroxide which comprises oxidase for the substrate to be determined, the chromogen represented by the formula (I) or (II) and peroxidase. The composition may contain buffer reagent.

Further, the composition may contain surfactants such as polyoxyethylenealkylether, antiseptics such as sodium azide, ascorbate oxidase for decomposing ascorbic acid, if necessary.

Further the composition may contain components necessary for producing hydrogen peroxide other than oxidase for the substrate.

Certain specific embodiments of the present invention are illustrated by the following representative examples.

## Example 1

In this example, 0.1M phosphate buffer solution (pH 6.0) containing 10 IU/ml peroxidase, 0.1 IU/ml uricase, 0.1 mg/ml compound II—6 and 0.1% Triton® X—100 is prepared as a test solution. To 3 ml of the test solution is added each of 20 µl of samples indicated in Table 3 and each mixture is incubated for reaction at 37°C with stirring for 10 minutes.

The absorbancy of each reaction solution at 665 nm is measured.

For comparison, the same procedures as described above are repeated except that 4AA-diethylaniline is used instead of compound II—6 and that the absorbancy of the reaction solution at 550 nm is measured. The results are shown in Table 3.

The following samples are referred to as $S_{1-4}$.

$S_1$ : 10 mg/dl uric acid standard solution

$S_2$ : normal human serum

$S_3$ : gouty patient serum

$S_4$ : deionized water

TABLE 3

| | the present method | | 4AA–diethylaniline | |
|---|---|---|---|---|
| | absorbancy | uric acid (mg/ml) | absorbancy | uric acid (mg/ml) |
| $S_1$ | 0.322 | (10.0) | 0.056 | (10.0) |
| $S_2$ | 0.157 | 4.6 | 0.026 | 4.4 |
| $S_3$ | 0.282 | 8.7 | 0.050 | 8.9 |
| $S_4$ | 0.014 | – | 0.002 | – |

As for the sample $S_2$, both the present method and the method using 4AA-diethylaniline are repeated 10 times and the absorbancy measured each time. The results are shown in Table 4.

TABLE 4

| | the present method | | 4AA–diethylaniline | |
|---|---|---|---|---|
| | absorbancy | uric acid (mg/dl) | absorbancy | uric acid (mg/dl) |
| 1 | 0.157 | 4.64 | 0.027 | 4.63 |
| 2 | 0.157 | 4.64 | 0.027 | 4.63 |
| 3 | 0.156 | 4.61 | 0.026 | 4.44 |
| 4 | 0.160 | 4.74 | 0.024 | 4.07 |
| 5 | 0.159 | 4.71 | 0.026 | 4.44 |
| 6 | 0.158 | 4.68 | 0.026 | 4.44 |
| 7 | 0.157 | 4.64 | 0.025 | 4.26 |
| 8 | 0.156 | 4.61 | 0.025 | 4.26 |
| 9 | 0.159 | 4.71 | 0.028 | 4.81 |
| 10 | 0.157 | 4.64 | 0.027 | 4.63 |
| the average | 0.157 | 4.66 | 0.026 | 4.46 |
| coefficient of variation | 0.81% | 0.92% | 4.35% | 4.72% |

As is apparent from Table 4, the coefficient of variation in the present method is very small and therefore the present method has good accuracy.

Example 2

The same procedures as described in Example 1 are repeated except that the chromogens indicated in Table 1 are used instead of compound II—6 and the enzymatic reactions with the normal serum are carried out. The absorbancy of each reaction solution is measured at $\lambda$ max for each chromogen and similar results to that with compound II—6 are obtained.

Example 3

The reagent solution is prepared by dissolving 10 mg of Compound II—6 in 100 ml of 0.1M phosphate buffer solution (pH 6.0) containing 15 mg of co-carboxylase, 200 IU of pyruvate oxidase, 0.1 ml of Triton® X—100 and 500 IU of peroxidase.

To three test tubes, each containing 3 ml of the reagent solution, is added (A) 0.05 ml of distilled water (B) 1 mg/dl pyruvic acid standard solution or (C) 0.05 ml of serum and each mixture is incubated at 37°C for 20 minutes. The absorbancy of each reaction solution is measured at 665 nm and the concentration of pyruvic acid in the serum is determined at 1.04 mg/dl.

Example 4

As the reagent solution, 100 ml of 0.05M phosphate buffer solution (pH 6.0) containing 0.1 ml of Triton® X—100, 100 U of peroxidase, 3.3 U of acyl Co A synthetase, 1.7 U of acyl-Co A oxidase, 33 μmol of Co enzyme A, 133 μmol of ATP, 133 μmol of magnesium chloride and 10 mg of compound II—4 is prepared.

To 3 ml of the reagent solution is added 0.02 ml of serum and the mixture is incubated at 37°C for 20 minutes. The absorbancy of the reaction solution is measured at 665 nm.

The standard curve is obtained by repeating the above procedures using a standard solution of palmitic acid and distilled water as a sample.

The acid content in the serum is calculated at 394 μ eg/l.

## Example 5

Reagent solution (1)

0.1 M phosphate buffer solution (pH 6.0) containing 20 mg/l hydrogen peroxide, 0.1 mg/ml compound II—6 and 0.1% Triton® X—100.

Reagent solution (2)

a reagent solution having the same composition as that of reagent solution (1) except that 0.1 mg/ml 4—AA and 0.2 mg/ml N,N-diethylaniline is used instead of compound II—6.

Reagent solution (3)

a solution having slight peroxidase activity.

Determination Procedures

3 ml of Reagent solution (1) is heated at 37°C for 10 minutes and 50 µl of Reagent solution (3) is added to the solution. One minute after the addition, the absorbancy of the mixture at 665 nm, which is defined as $E_1$ is measured. Further, the absorbancy which is measured 5 minutes after the addition, is defined as $E_5$.

As for the reagent solution (2), a similar reaction is carried out to obtain the absorbancies at 550 nm one minute after the addition and 5 minutes after the addition, which are defined as $E_1'$ and $E_5'$, respectively. The result is that the value of $E_5—E_1$ is 6.2 times as much as that of $E_5'—E_1'$. Therefore it is concluded that the present method has good sensitivity.

## Example 6

In this example, one g of Methylene Blue (produced by Wako Junyaku Co., Ltd.) is dissolved in 100 ml of water and one g of sodium salt of hydrogen borohydride is added to the solution portionwise. The mixture is subjected to reaction at 20°C.

When a precipitate is formed and the reaction solution is discoloured, 20 ml of chloroform is added thereto and the mixture is vigorously stirred. The chloroform layer is separated off and one g of sodium sulfate anhydride is added to the chloroform layer residue. The mixture is stirred, filtered, dehydrated and desalted.

To the filtrate is added 2 ml of phenyl isocyanate and the mixture is reacted with stirring at room temperature for 16 hours. To the reaction mixture is added methanol and the mixture is stirred for 3 hours to decompose excess isocyanate.

The reaction mixture is charged on a column packed with silica gel having a size of 60—80 mesh and elution is carried out with chloroform.

The eluate is concentrated to dryness under reduced pressure to obtain 1.3 g of compound II—9, as a white or light brown amorphous powder.

The melting point of the product is 143—145°C and the infrared spectrum and NMR spectrum are shown in Figures 9 and 18, respectively.

## Example 7

The same procedures as described in Example 6 are repeated except that the compounds indicated in Table 5 are used instead of phenylisocyanate to obtain the desired compounds shown in Table 5.

TABLE 5

| The desired compound | Isocyanates (g) | Yield (g) | M.P. (°C) | IR Fig. No. | NMR Fig. No. |
|---|---|---|---|---|---|
| II—4 | hexamethylene diisocyanate (0.8) | 0.61 | 96—99 | 4 | 13 |
| II—5 | tolylene 2,4-diisocyanate (0.9) | 0.79 | 155—157 | 5 | 14 |
| II—6 | m-xylylene diisocyanate (0.9) | 0.59 | 96—100 | 6 | 15 |
| II—7 | 1-naphthylisocyanate (0.8) | 0.84 | 114—117 | 7 | 16 |
| II—8 | acetyl chloride (0.2) | 0.56 | 178—180 | 8 | 17 |

Example 8

In this example, one g of Bindschedler's Green Leuco Base (Dotite BG, produced by Dojin Yakukagaku Kenkyusho) is dissolved in 20 ml of chloroform. Each of the compounds (isocyanates) indicated in Table 6 is added to the solution and each mixture is subjected to reaction at room temperature for 16 hours. Methanol is added to each reaction mixture to decompose the unreacted isocyanate and the same purification procedures as in Example 6 are repeated to obtain the desired compounds shown in Table 6.

TABLE 6

| Desired compound | Isocyanates | Yield (g) | M.P. (°C) | IR Fig. No. | NMR Fig. No. |
|---|---|---|---|---|---|
| I—1 | Methyl isothiocyanate | 0.72 | 180–182 | 1 | 11 |
| I—2 | Cyclohexyl-isocyanate | 0.88 | 133–135 | 2 | 12 |
| I—3 | 3,4-dichloroisocyanate | 1.23 | 207–208 | 3 | – |

Example 9

A similar procedure to that of Example 5 is carried out except that Meldola Blue is used instead of Methylene Blue to obtain compound II—10 in an oily form.

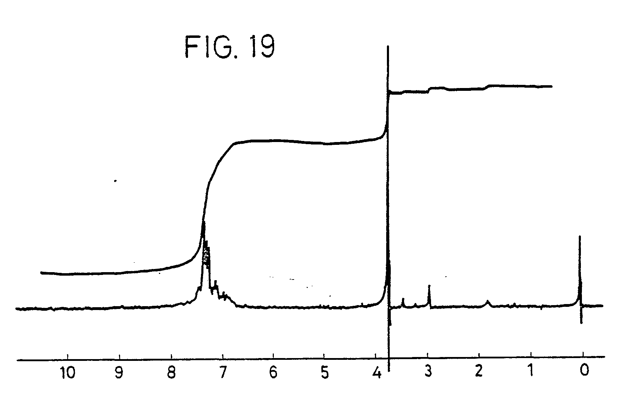

The infrared spectrum and NMR spectrum of the desired compound are shown in Figs. 10 and 19, respectively.

Brief explanation of the figures of the drawings

The infrared absorption spectra of compounds I—1 to 3 and II—4 to 10 are shown in Figures 1 to 10.

NMR spectra in $CDCl_3$ of compounds I—1 to 2 and II—4 to 10 are shown in Figures 11 to 19.

**Claims**

1. A method for the determination of hydrogen peroxide in a sample which comprises reacting the hydrogen peroxide with peroxidase in the presence of a chromogen and measuring the absorbancy of the reaction solution in the visible ray region, characterised in that the chromogen used is a compound of the formula

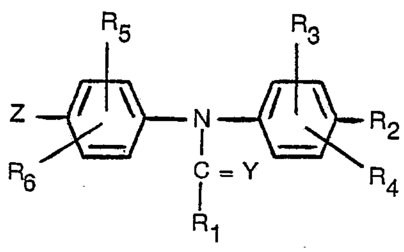

[I]

or

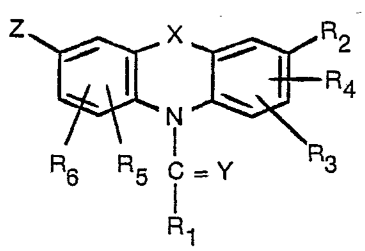

[II]

wherein

Z represents hydroxyl, amino or substituted amino wherein the substituent(s) is or are selected from $C_1$—$C_4$ alkyl, $C_2$—$C_5$ alkenyl, hydroxy($C_1$—$C_4$)alkyl, ($C_2$—$C_5$)acyl-substituted ($C_1$—$C_4$)alkyl, phenyl, benzyl, naphthyl, or substituted phenyl, being a phenyl group containing up to five substituents selected from $C_1$—$C_4$ alkyl, halogen, amino, ($C_1$—$C_4$)alkoxycarbonylamino, ($C_1$—$C_4$)alkoxycarbonylamino($C_1$—$C_4$)alkyl, $C_1$—$C_4$ alkoxy or $C_2$—$C_5$ acyl;

$R_1$ represents hydrogen, $C_1$—$C_4$ alkyl, $C_2$—$C_5$ alkenyl, phenyl, benzyl, naphthyl, substituted phenyl (as defined under Z), amino or mono-substituted amino in which the substituent is $C_1$—$C_4$ alkyl, $C_2$—$C_5$ alkenyl, phenyl, naphthyl, substituted phenyl (as defined under Z) or ($C_1$—$C_4$)alkyl substituted by $C_1$—$C_4$ alkoxy-carbonylamino, phenyl or substituted phenyl as defined under Z;

$R_2$ represents hydrogen, hydroxyl, $C_1$—$C_4$ alkyl, $C_2$—$C_5$ alkenyl, amino, ($C_1$—$C_4$)alkylamino, $C_1$—$C_4$ alkoxy, phenyl, benzyl, naphthyl or substituted phenyl as defined under Z;

$R_3$, $R_4$, $R_5$ and $R_6$, when taken separately represent hydrogen, $C_1$—$C_4$ alkyl, $C_2$—$C_5$ alkenyl, $C_2$—$C_5$ acyl, $C_1$—$C_4$ alkoxy, hydroxy ($C_1$—$C_4$) alkyl, halogen, sulfo, nitro, carboxyl, hydroxyl, phenyl, benzyl, naphthyl or substituted phenyl as defined under Z;

or when taken together, $R_3$ and $R_4$ or $R_5$ and $R_6$ may each represent $C_3$—$C_4$ alkenylene;

X represents –S–, –O–, 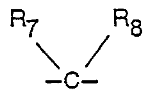 or 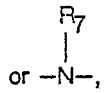,

where $R_7$ and $R_8$ represent hydrogen $C_1$—$C_4$ alkyl, $C_2$—$C_5$ alkenyl, phenyl, benzyl, naphthyl or substituted phenyl as defined under Z; and

Y is oxygen or sulfur.

2. A method according to claim 1, wherein Z is dimethylamino.

3. A method according to claim 1 or 2 wherein $R_2$ is dimethylamino.

4. A method according to claim 1 wherein both Z and $R_2$ are dimethylamino, $R_1$ is a mono-substituted amino group in which the substituent is a substituent group as listed in claim 1 under the corresponding group as defined under Z, and $R_3$, $R_4$, $R_5$ and $R_6$ are hydrogen.

5. A method according to any preceding claim, wherein said hydrogen peroxide is a product formed by an enzymatic reaction.

6. A method according to claim 5, wherein said enzymatic reaction is an oxidation reaction of a substrate using oxidase.

7. A method according to claim 6, wherein said oxidase is selected from uricase, cholesterol oxidase, xanthin oxidase, choline oxidase, pyruvate oxidase, glycerin triphosphate oxidase, acyl Co A oxidase, glycerol oxidase, glucose oxidase and galactose oxidase.

8. A method according to claim 5, 6 or 7, wherein said hydrogen peroxide-producing reaction and the reaction of hydrogen peroxide with chromogen are conducted simultaneously.

9. A method according to any preceding claim, wherein said reaction is carried out in a buffer solution.

10. A test composition for the determination of hydrogen peroxide which comprises the chromogen defined in claim 1 and peroxidase.

11. A test composition according to claim 10, wherein said composition contains a buffer.

12. A test composition according to claim 10 and 11, wherein said composition contains a surfactant, an antiseptic or ascorbate oxidase.

13. A test composition according to claim 10, 11 or 12, wherein said composition further contains an oxidase-containing hydrogen peroxide-producing system.

**Revendications**

1. Procédé pour la détermination du peroxyde d'hydrogène dans un échantillon, qui comprend la réaction du peroxyde d'hydrogène avec la peroxydase en présence d'un chromogène et la mesure de l'absorbance de la solution réactionnelle dans la région visible du spectre, caractérisé par le fait que le chromogène utilisé est un composé de formule

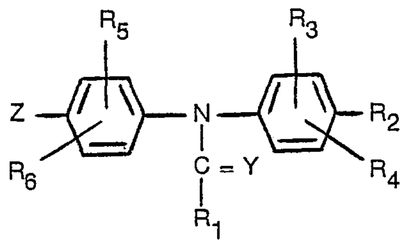

[I]

ou

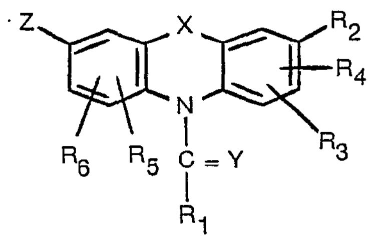

[II]

dans lesquelles Z représente un groupe hydroxyle, amino ou amino substitué où, dans ce cas, on choisit le ou les substituants parmi les groupes alkyle en $C_1$—$C_4$, alcényle en $C_2$—$C_5$, hydroxyalkyle en $C_1$—$C_4$, acyl($C_2$—$C_5$)-alkyle($C_1$—$C_4$), phényle, benzyle, naphtyle ou phényle substitué, ce dernier étant un groupe phényle contenant jusqu'à cinq substituants choisis parmi un groupe alkyle en $C_1$—$C_4$, un halogène, un groupe amino, alcoxy($C_1$—$C_4$)carbonylamino, alcoxy($C_1$—$C_4$)-carbonylamino-alkyle($C_1$—$C_4$), alcoxy en $C_1$—$C_4$ ou un groupe acyle en $C_2$—$C_5$;

$R_1$ représente un atome d'hydrogène, un groupe alkyle en $C_1$—$C_4$, un groupe alcényle en $C_2$—$C_5$, un groupe phényle, benzyle, naphtyle, phényle substitué (tel qu'on l'a défini pour Z), amino ou amino monosubstitué dans lequel le substituant est un groupe alkyle en $C_1$—$C_4$, un groupe alcényle en $C_2$—$C_5$, phényle, naphtyle, phényle substitué (tel qu'on l'a défini pour Z) ou un groupe alkyle en $C_1$—$C_4$ substitué avec un groupe alcoxy($C_1$—$C_4$)-carbonylamino, un groupe phényle ou un groupe phényle substitué comme ln l'a indiqué pour Z;

$R_2$ représente un atome d'hydrogène, un groupe hydroxyle, alkyle en $C_1$—$C_4$, alcényle en $C_2$—$C_5$, amino, alkylamino en $C_1$—$C_4$, alcoxy en $C_1$—$C_4$, phényle, benzyle, naphtyle ou un groupe phényle substitué tel qu'on l'a défini pour Z;

$R_3$, $R_4$, $R_5$ et $R_6$, s'ils sont considérés isolément, représentent un atome d'hydrogène, un groupe alkyle en $C_1$—$C_4$, un groupe alcényle en $C_2$—$C_5$, un groupe acyle en $C_2$—$C_5$, un groupe alcoxy en $C_1$—$C_4$, un groupe hydroxy-alkyle en $C_1$—$C_4$, un halogène, un groupe sulfo, nitro, carboxyle, hydroxyle, phényle, benzyle, naphtyle ou un groupe phényle substitué tel qu'on l'a défini pour Z; ou, s'ils sont considérés ensemble, $R_3$ et $R_4$ ou $r_5$ et $R_6$ peuvent chacun représenter un groupe alkenylène en $C_3$—$C_4$;

X représente —S—, —O—, 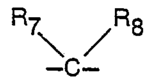 ou 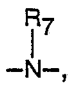,

où $R_7$ et $R_8$ représentent un atome d'hydrogène, un groupe alkyle en $C_1$—$C_4$, un groupe alcényle en $C_2$—$C_5$, un groupe phényle, benzyle, naphtyle ou un groupe phényle substitué tel qu'on l'a défini pour Z;
et Y est un atome d'oxygène ou de soufre.

2. Procédé selon la revendication 1, dans lequel Z est constitué par un groupe diméthylamino.

3. Procédé selon l'une des revendications 1 ou 2, dans lequel $R_2$ est constitué par un groupe diméthylamino.

4. Procédé selon la revendication 1, dans lequel Z et $R_2$ sont des groupes diméthylamino; $R_1$ est un groupe amino monosubstitué dans lequel le substituant est un des groupes substituants cités dans la revendication 1, dans la liste des groupes correspondants pour Z, et $R_3$, $R_4$, $R_5$ et $R_6$ sont des atomes d'hydrogène.

5. Procédé selon l'une quelconque des revendications précédentes, dans lequel ledit peroxyde d'hydrogène est un produit formé par une réaction enzymatique.

6. Procédé selon la revendication 5, dans lequel ladite réaction enzymatique est une réaction d'oxydation d'un substrat utilisant une oxydase.

7. Procédé selon la revendication 6, dans lequel ladite oxydase est choisie parmi l'uricase, la cholestérol-oxydase, la xanthineoxydase, la choline-oxydase, la pyruvate-oxydase, la glycérine-triphosphate-oxydase, l'acyl-co A-oxydase, la glycérol-oxydase, la glucose-oxydase et la galactose-oxydase.

8. Procédé selon la revendication 5, 6 ou 7, dans lequel ladite réaction produisant du peroxyde d'hydrogène et la réaction du peroxyde d'hydrogène avec le chromogène sont effectuées simultanément.

9. Procédé selon l'une quelconque des revendications précédentes, dans lequel ladite réaction est mise en oeuvre dans une solution tampon.

10. Composition d'essai pour la détermination du peroxyde d'hydrogène qui comprend le chromogène défini dans la revendication 1 et de la peroxydase.

11. Composition d'essai selon la revendication 10, dans laquelle ladite composition contient un tampon.

12. Composition d'essai selon la revendication 10 ou 11, dans laquelle ladite composition contient une substance tensio-active, un antiseptique ou de l'ascorbate-oxydase.

13. Composition d'essai selon la revendication 10, 11 ou 12, dans laquelle ladite composition contient en outre un système producteur de peroxyde d'hydrogène contenant une oxydase.

**Patentansprüche**

1. Verfahren zur Bestimmung von Wasserstoffperoxid in einer Probe, umfassend die Umsetzung von Wasserstoffperoxid mit Peroxidase in Gegenwart eines Chromogen und Messen der Absorption der Reaktionslösung im Bereich sichtbaren Lichts, dadurch gekennzeichnet, daß das verwendete Chromogen eine Verbindung der Formel ist

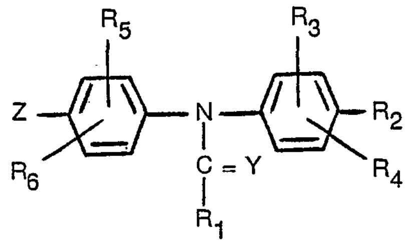

[I]

oder

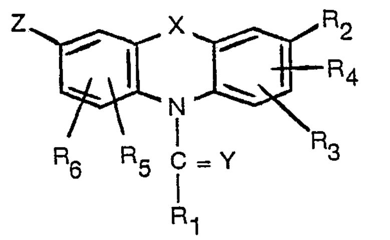

[II]

wobei Z eine Hydroxyl-, Amino- oder substituierte Aminogruppe darstellt, in der der (die) Substituent(en) (ein) $C_1$—$C_4$-Alkyl-, $C_2$—$C_5$-Alkenyl-, Hydroxy($C_1$—$C_4$)-alkyl-, ($C_2$—$C_5$)-Acyl($C_1$—$C_4$)-alkyl-, Phenyl-, Benzyl-, Naphthyl- oder substituierte(r) Phenylrest(e), welcher ein Phenylrest mit bis zu 5 Substituenten, ausgewählt aud $C_1$—$C_4$-Alkyl-, Halogen-, Amino-, ($C_1$—$C_4$)-Alkoxycarbonylamino-, ($C_1$—$C_4$)-Alkoxycarbonylamino($C_1$—$C_4$)-alkyl-, $C_1$—$C_4$-Alkoxy- oder $C_2$—$C_5$-Acylresten, ist (sind);

$R_1$ ein Wasserstoffatom, einen $C_1$—$C_4$-Alkyl-, $C_2$—$C_5$-Alkenyl-, Phenyl-, Benzyl-, Naphthyl-, substituierten Phenyl (definiert wie bei Z), Amino- oder monosubstituierten Aminorest darstellt, in dem der Substituent ein $C_1$—$C_4$-Alkyl-, $C_2$—$C_5$-Alkenyl-, Phenyl-, Naphthyl-, substituierter Phenyl- (de-finiert wie bei Z) oder ($C_1$—$C_4$)-Alkylrest, substituiert durch einen $C_1$—$C_4$-Alkoxycarbonylamino-, Phenyl- oder substituierten Phenylrest, wie bei Z definiert, ist;

$R_2$ ein Wasserstoffatom, eine Hydroxyl-, $C_1$—$C_4$-Alkyl-, $C_2$—$C_5$-Alkenyl-, Amino-, ($C_1$—$C_4$)-Alkylamino-, $C_1$—$C_4$-Alkoxy-, Phenyl-, Benzyl-, Naphthyl- oder substituierte Phenylgruppe, wie bei Z definiert, darstellt;

$R_3$, $R_4$, $R_5$ und $R_6$ Wasserstoffatome, $C_1$—$C_4$-Alkyl-, $C_2$—$C_5$-Alkenyl-, $C_2$—$C_5$-Acyl-, $C_1$—$C_4$-Alkoxy-, Hydroxy-($C_1$—$C_4$)-alkyl-, Halogen-, Sulfo-, Nitro-, Carboxyl-, Hydroxyl-, Phenyl-, Benzyl-, Naphthyl- oder substituierte Phenylgruppen, wie bei Z definiert, bedeuten, wenn sie einzeln vorliegen, oder $R_3$ und $R_4$ oder $R_5$ und $R_6$ zusammengenommen jeweils einen $C_3$—$C_4$-Alkenylenrest darstellen können;

X —S—, —O—, 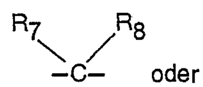 oder 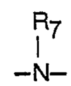 bedeutet,

wobei $R_7$ und $R_8$ Wasserstoffatome, $C_1$—$C_4$-Alkyl-, $C_2$—$C_5$-Alkenyl-, Phenyl-, Benzyl-, Naphthyl- oder substituierte Phenylreste, wie bei Z definiert, bedeuten; und

Y ein Sauerstoff- oder Schwefelatom ist.

2. Verfahren nach Anspruch 1, wobei Z eine Dimethylaminogruppe ist.

3. Verfahren nach Anspruch 1 oder 2, wobei $R_2$ eine Dimethylaminogruppe ist.

4. Verfahren nach Anspruch 1, wobei sowohl Z als auch $R_2$ Dimethylaminogruppen sind, $R_1$ eine monosubstituierte Aminogruppe ist, in der der Substituent eine Substituentengruppe nach der Aufzählung im Anspruch 1 unter der entsprechenden Gruppe wie bei Z definiert ist, und $R_3$, $R_4$, $R_5$ und $R_6$ Wasserstoffatome sind.

5. Verfahren nach einem der vorangehenden Ansprüche, wobei das Wasserstoffperoxid ein durch eine enzymatische Reaktion gebildetes Produkt ist.

6. Verfahren nach Anspruch 5, wobei die enzymatische Reaktion eine Oxidationsreaktion eines Substrats unter Verwendung von Oxidase ist.

7. Verfahren nach Anspruch 6, wobei die Oxidase Uricase, Cholesterin-oxidase, Xanthin-oxidase, Cholin-oxidase, Pyruvat-oxidase, Glycerintriphosphatoxidase, Acyl Co A-oxidase, Glycerin-oxidase, Glucoseoxidase oder Galactose-oxidase ist.

8. Verfahren nach Anspruch 5, 6 oder 7, wobei die Wasserstoffperoxid-erzeugende Umsetzung und die Umsetzung von Wasserstoffperoxid mit dem Chromogen gleichzeitig durchgeführt werden.

9. Verfahren nach einem der vorangehenden Ansprüche, wobei die Umsetzung in einer Pufferlösung durchgeführt wird.

10. Testsatz zur Bestimmung von Wasserstoffperoxid, enthaltend das Chromogen gemäß Anspruch 1 und Peroxidase.

11. Testsatz nach Anspruch 10, wobei der Satz einen Puffer enthält.

12. Testsatz nach Anspruch 10 oder 11, wobei der Satz ein grenzflächenaktives Mittel, ein antiseptisches Mittel oder Ascorbat-oxidase enthält.

13. Testsatz nach Anspruch 10, 11 oder 12, wobei der Satz ferner ein Oxidase enthaltendes, Wasserstoffperoxid erzeugendes System enthält.

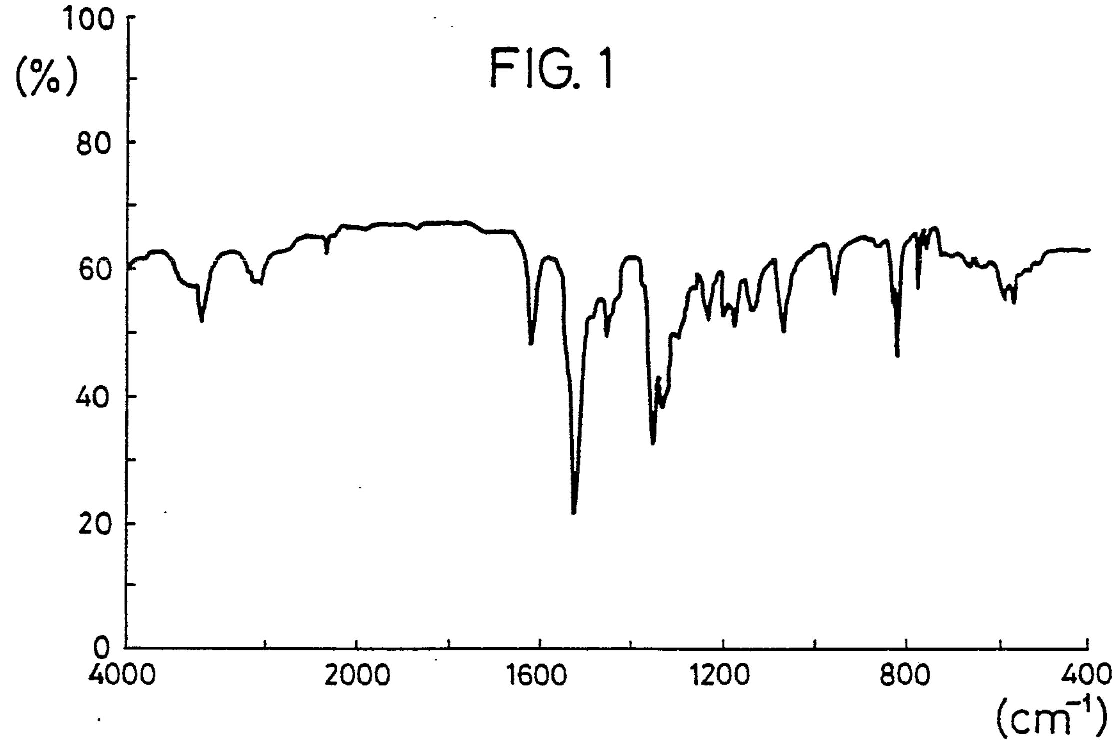
100
(%)
80
60
40
20
0
4000
2000
1600
1200
800
400
(cm⁻¹)

FIG. 1

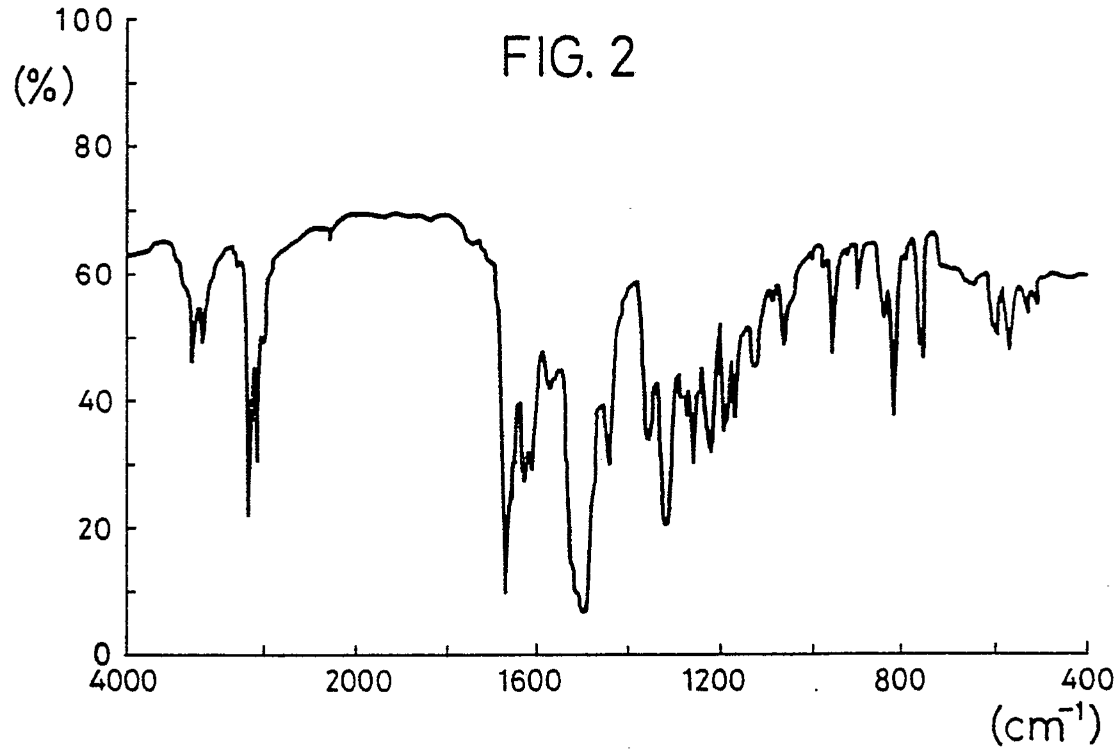
100
(%)
80
60
40
20
0
4000
2000
1600
1200
800
400
(cm⁻¹)

FIG. 2

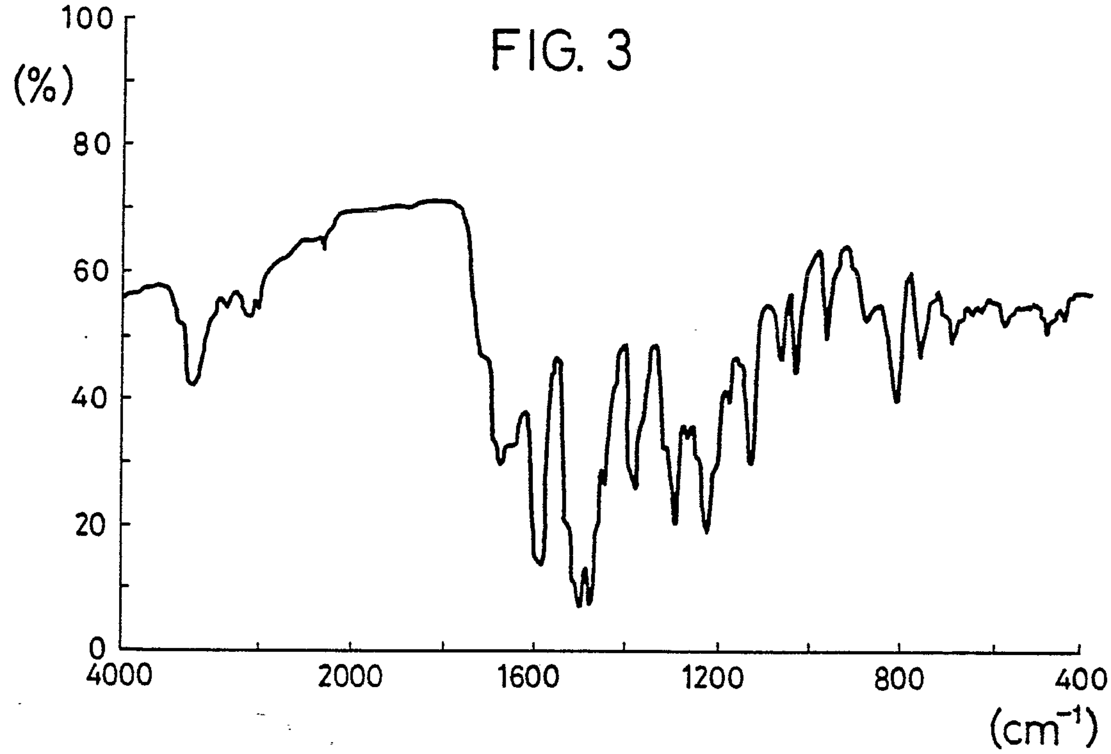
100
(%)
80
60
40
20
0
4000
2000
1600
1200
800
400
(cm⁻¹)

FIG. 3

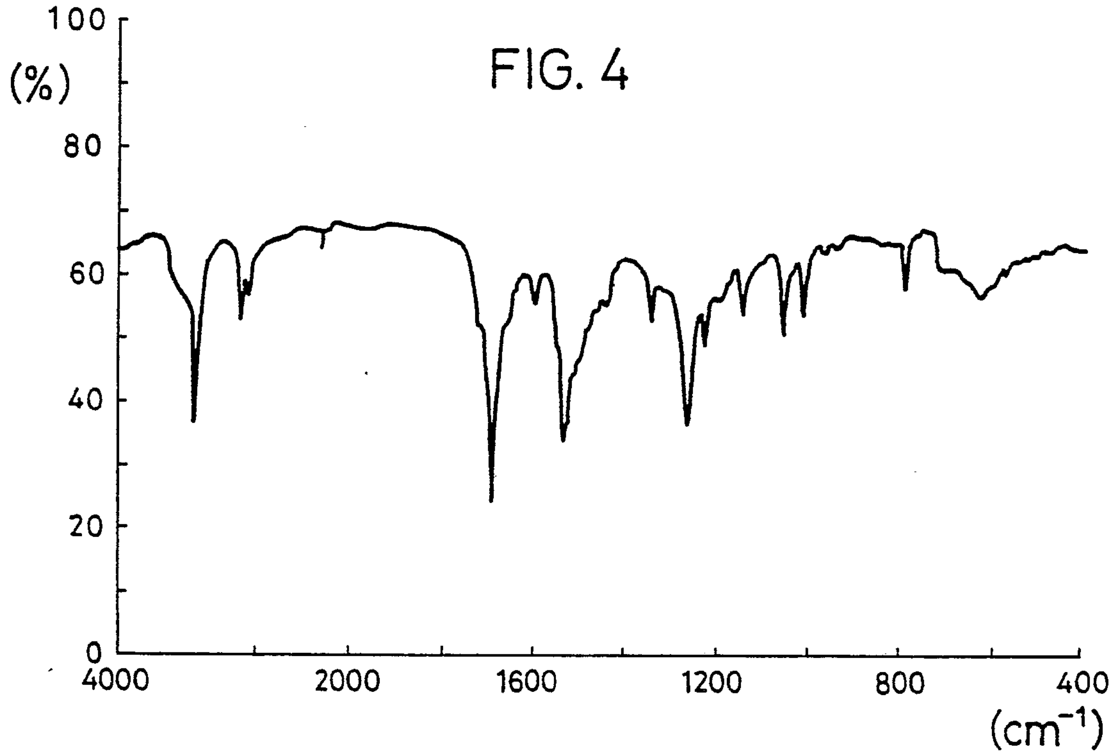
100
(%)
80
60
40
20
0
4000
2000
1600
1200
800
400
(cm⁻¹)

FIG. 4

FIG. 5

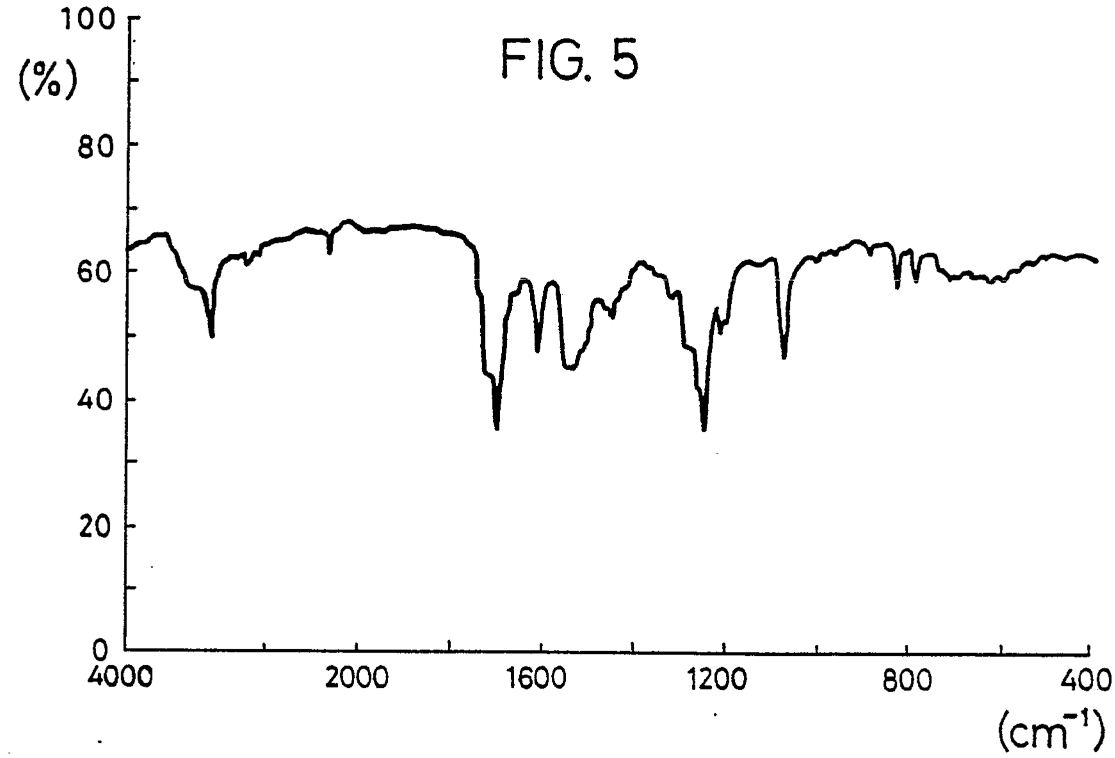
100
(%)
80
60
40
20
0
4000
2000
1600
1200
800
400
(cm⁻¹)

FIG. 6

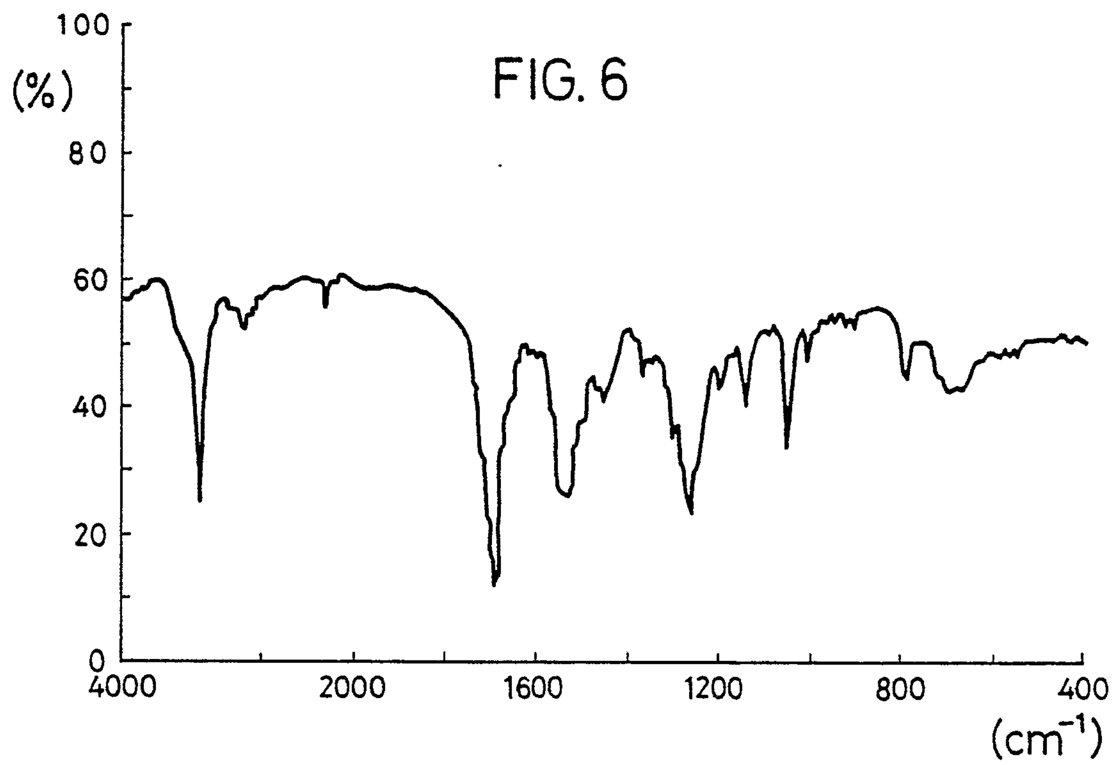
100
(%)
80
60
40
20
0
4000
2000
1600
1200
800
400
(cm⁻¹)

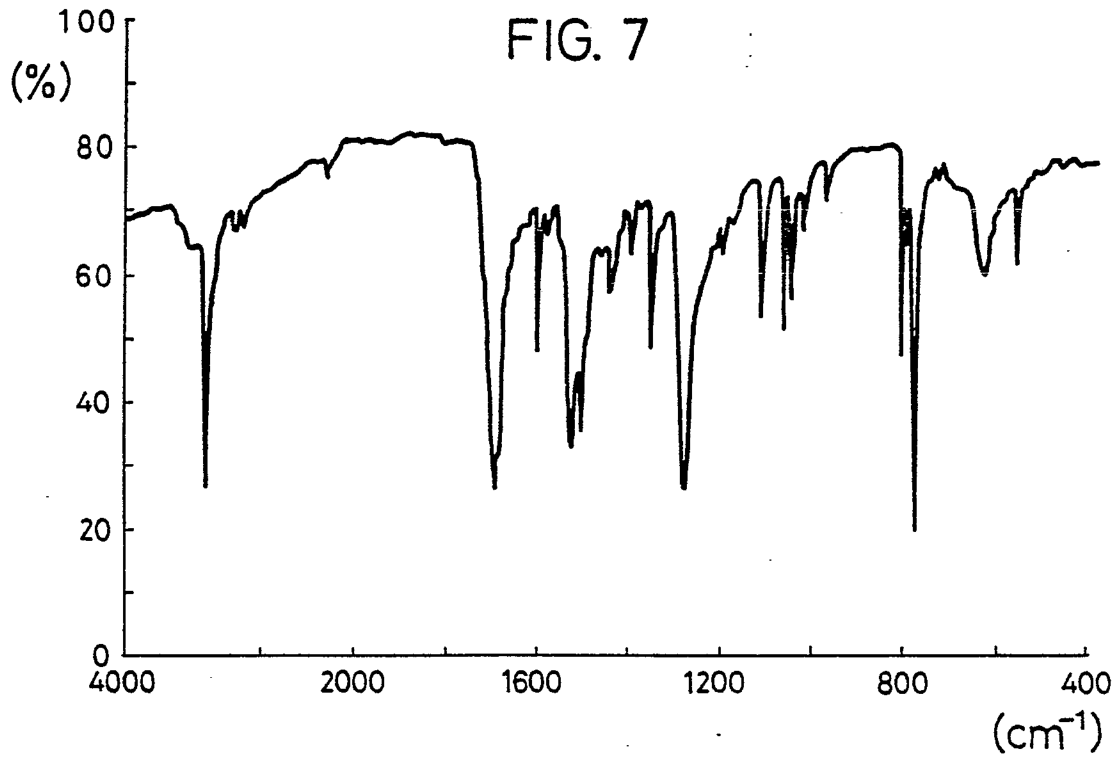
100
(%)
80
60
40
20
0
4000
2000
1600
1200
800
400
(cm$^{-1}$)

FIG. 7

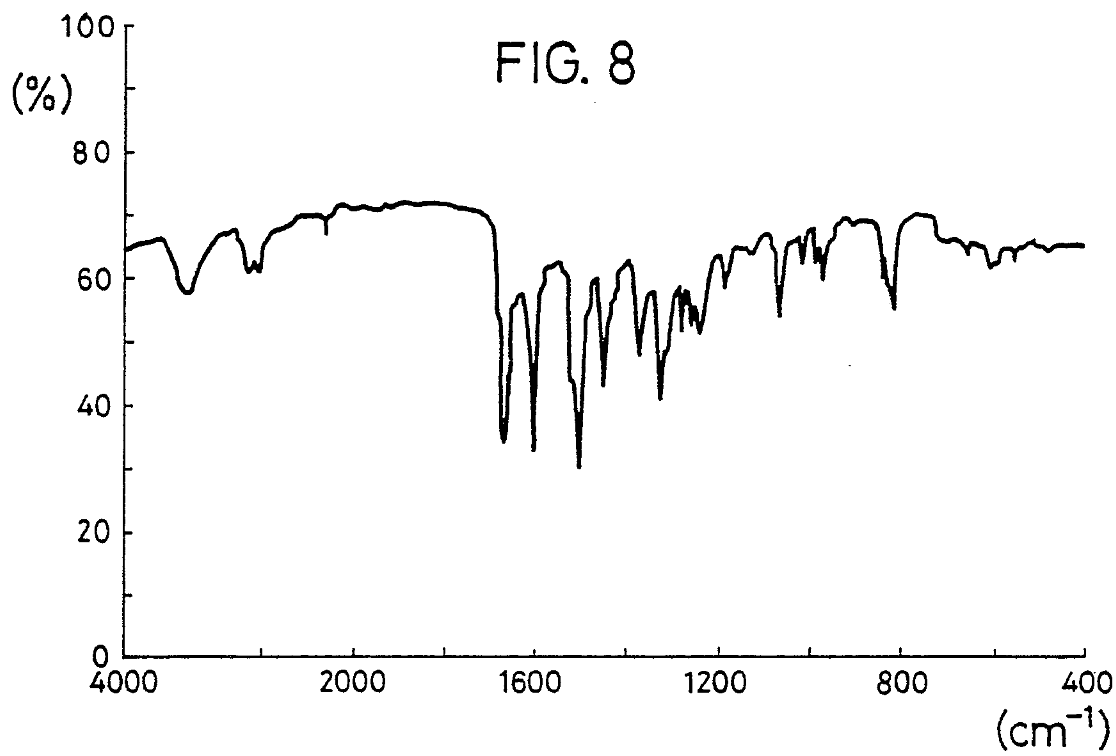
100
(%)
80
60
40
20
0
4000
2000
1600
1200
800
400
(cm$^{-1}$)

FIG. 8

FIG. 9

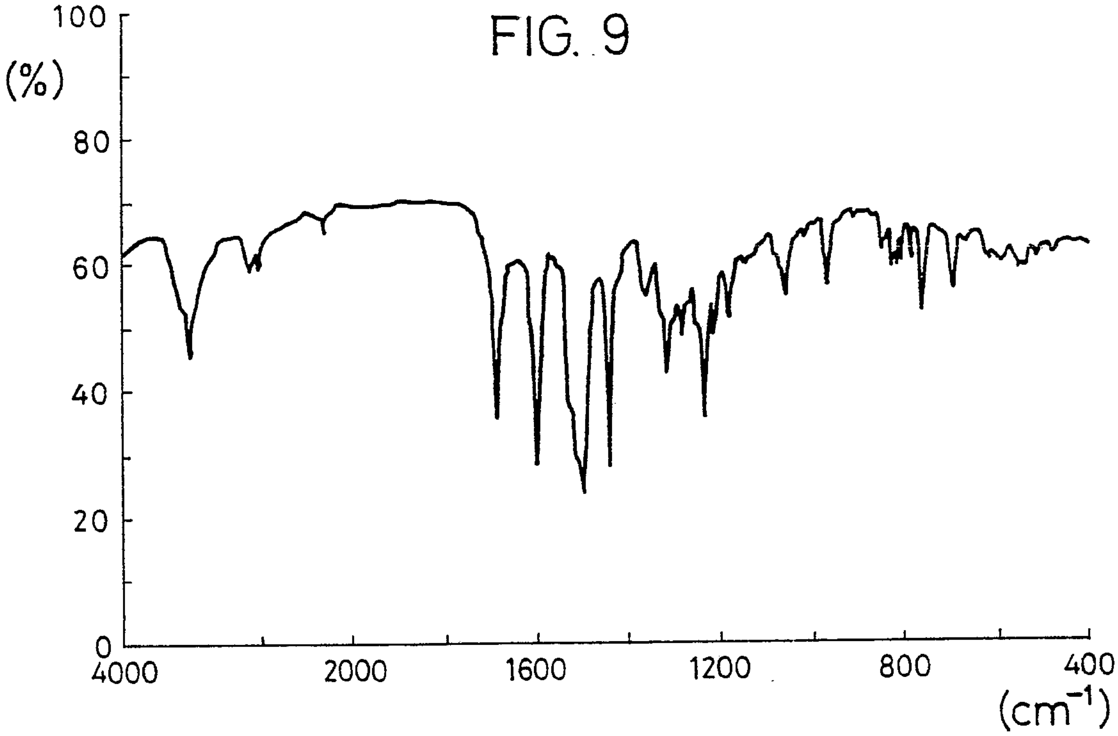
100
(%)
80
60
40
20
0
4000
2000
1600
1200
800
400
(cm$^{-1}$)

FIG. 10

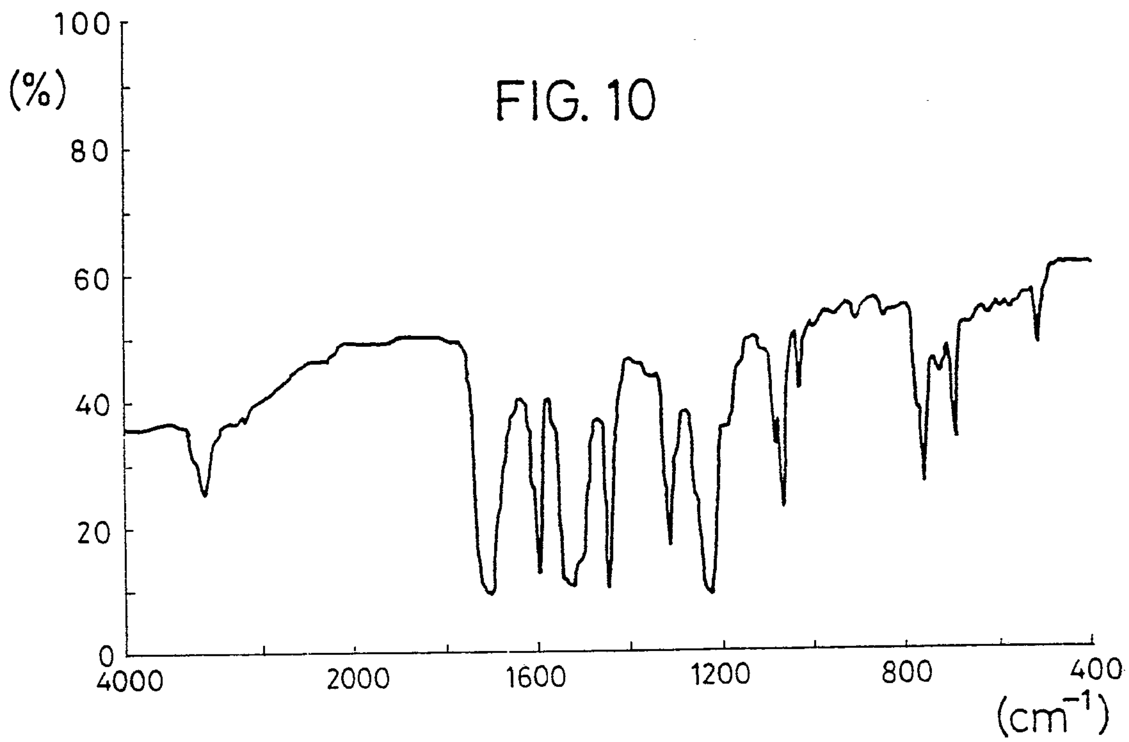
100
(%)
80
60
40
20
0
4000
2000
1600
1200
800
400
(cm$^{-1}$)

FIG. 11

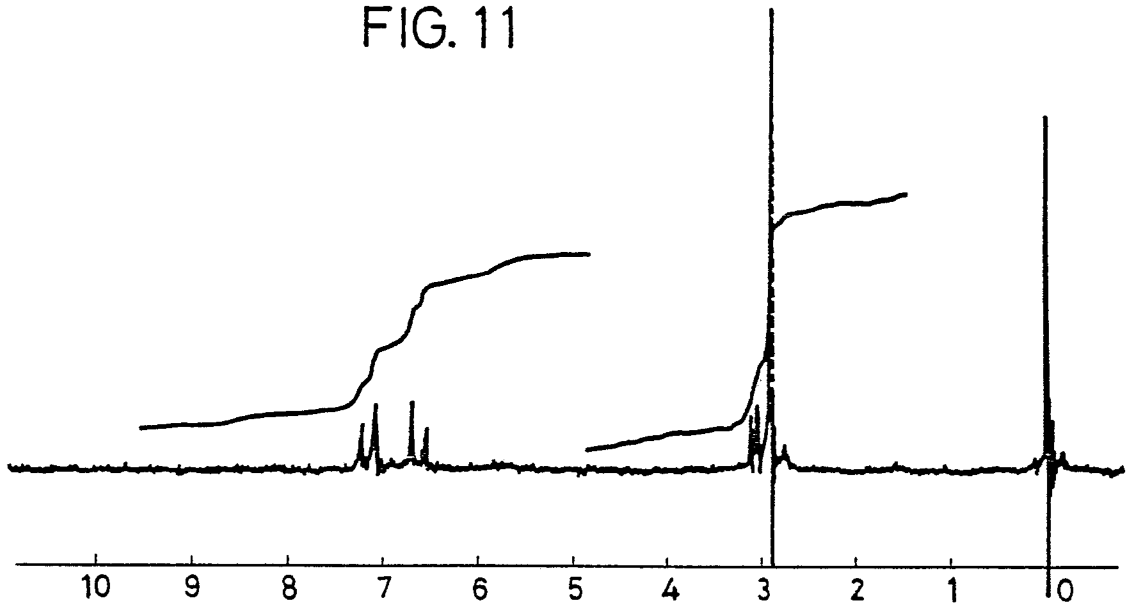


FIG. 12

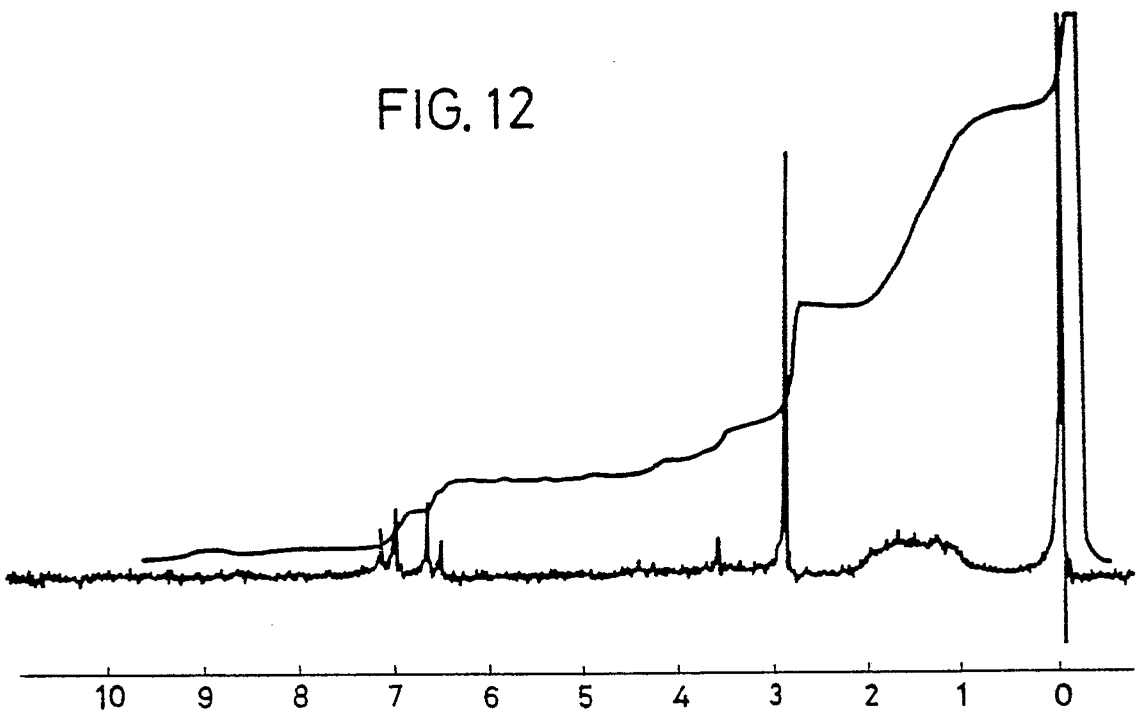

FIG. 13

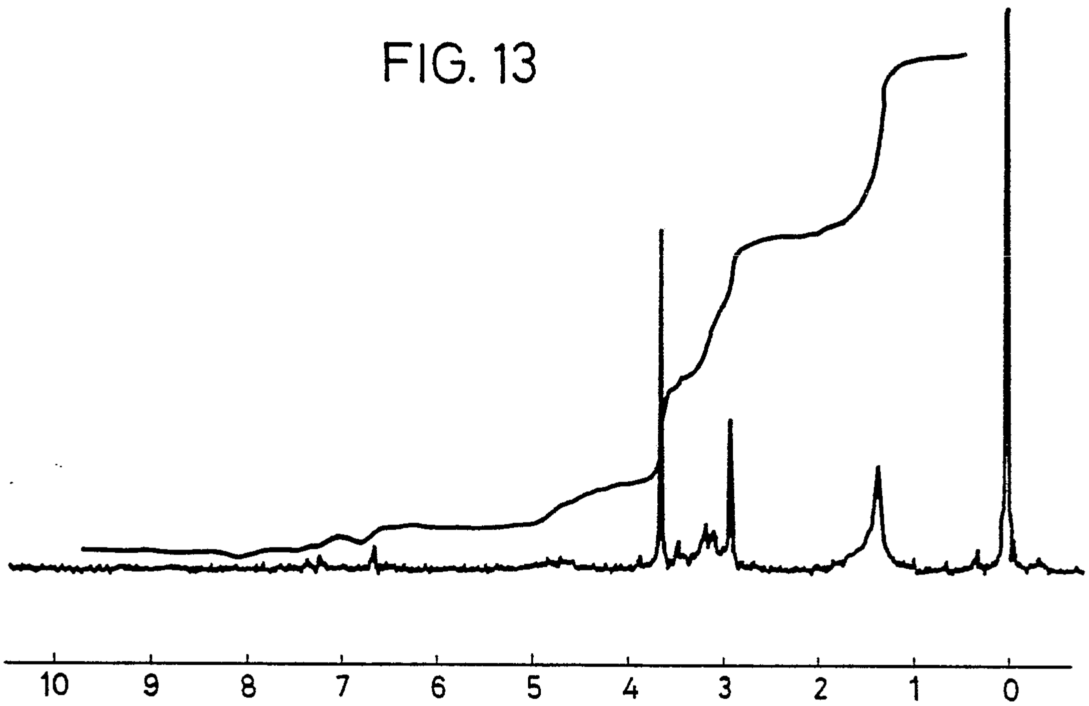


FIG. 14

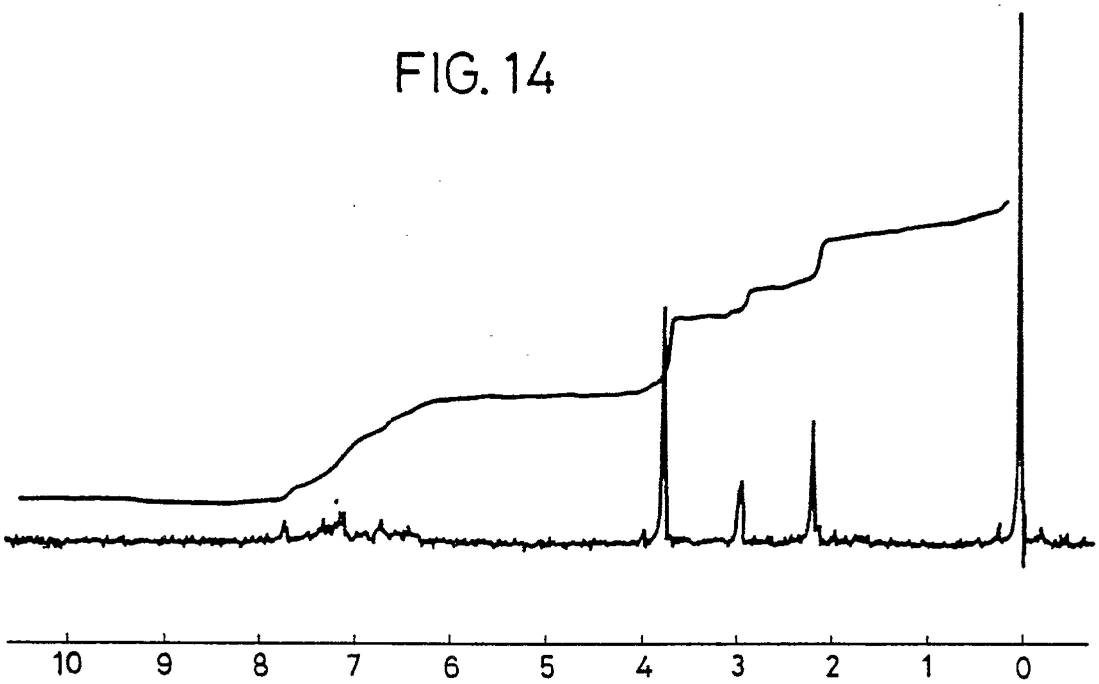

FIG. 15

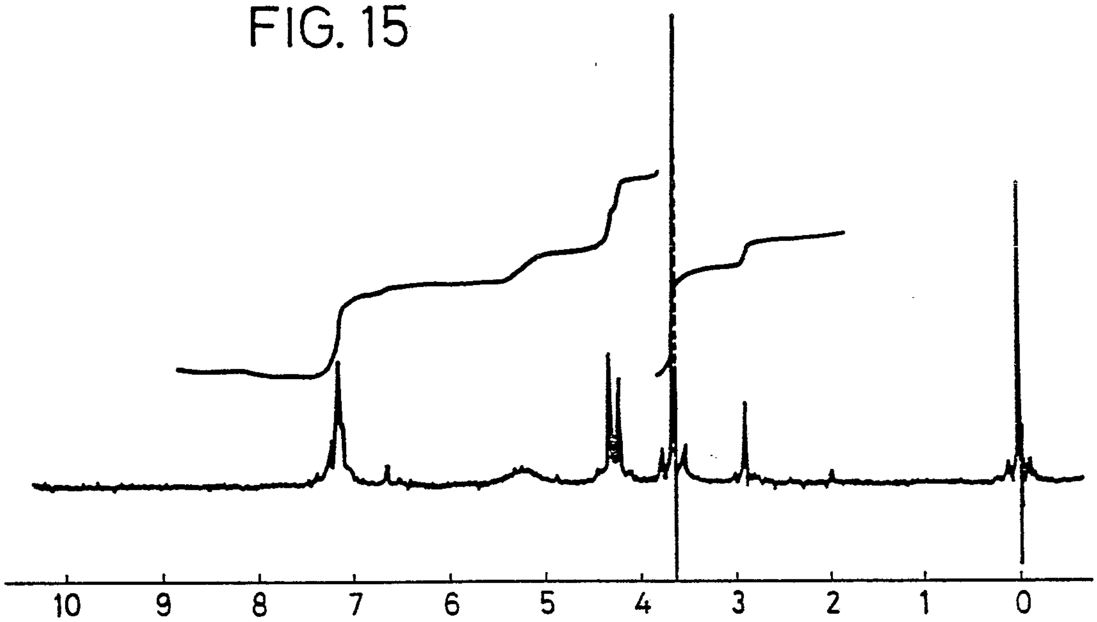


FIG. 16

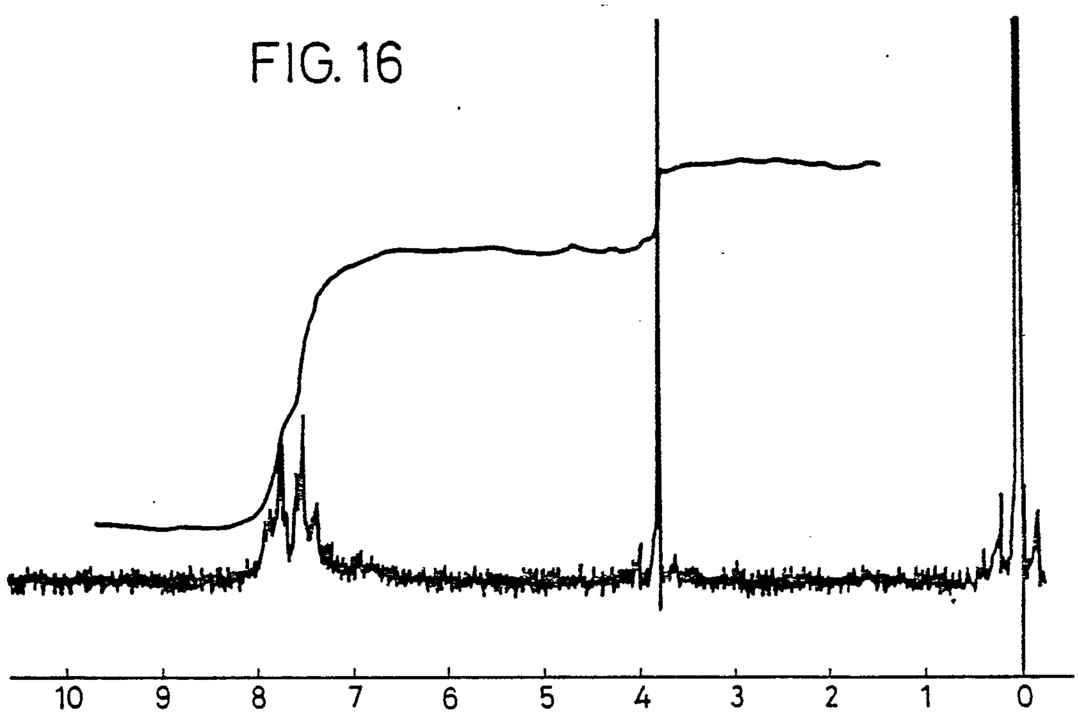

FIG. 17

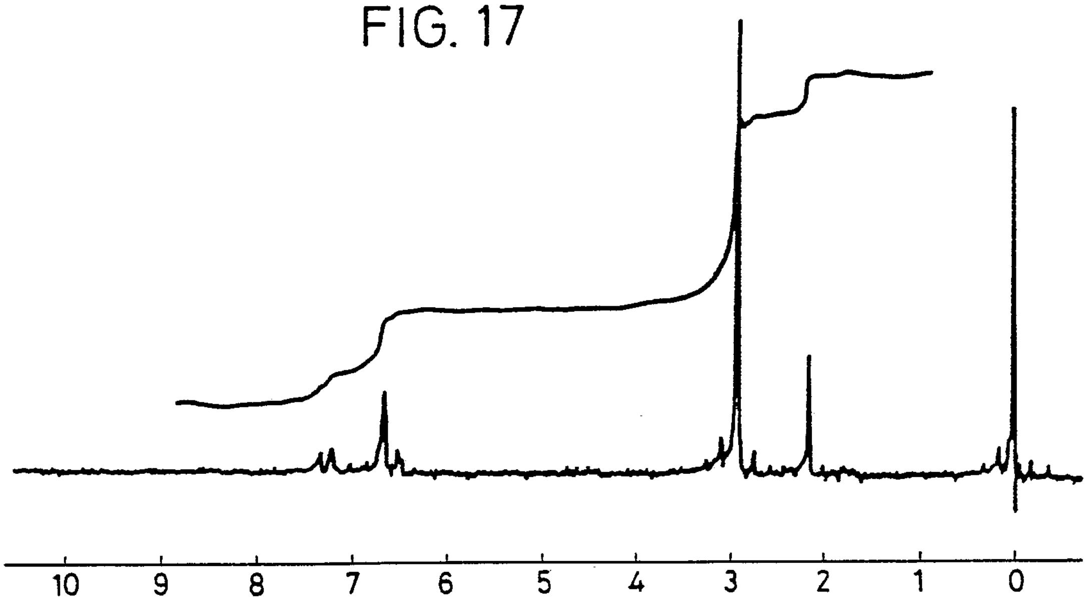


FIG. 18

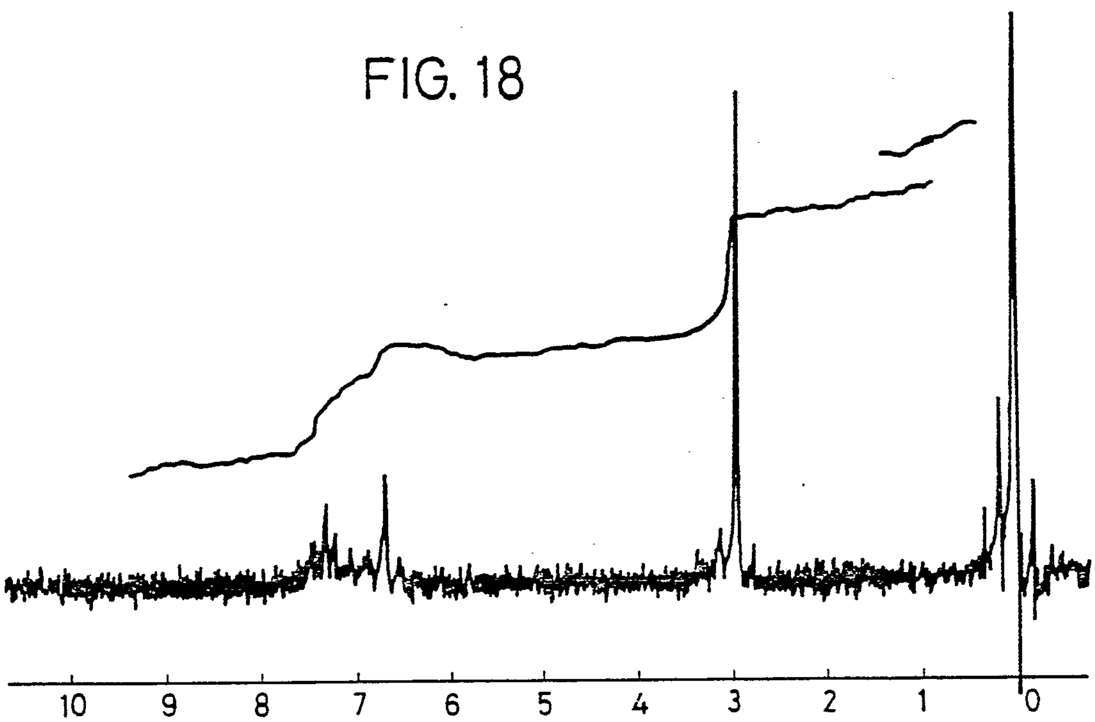

FIG. 19